# EUROPEAN PATENT SPECIFICATION

(11) **EP 1 344 066 B1**
(45) Date of publication and mention of the grant of the patent: **12.08.2009**
(21) Application number: 01994446.1
(22) Date of filing: 19.12.2001
(51) Int. Cl.: G01N 33/86, C07K 14/745

(54) **PROTEIN S FUNCTIONAL ASSAY**
FUNKTIONELLES ASSAY FÜR PROTEIN S
ESSAI FONCTIONNEL DE PROTEINE S

(30) Priority: 19.12.2000 US 256703 P
(43) Date of publication of application: 17.09.2003
(73) Proprietor: Instrumentation Laboratory Company, Bedford, MA 01730 (US)
(72) Inventor: DAI, Yong, Mechanicsville, VA 23111 (US); Biqing Ye, Orangeburg, NY 10962 (US); CHEN, Kui, Raleigh, NC 27613 (US); BRUGUERA, Pau, Lexington, MA 02421 (US); TANG, ShaMay, Bardonia, NY 10954 (US); LAWSON, Daniel, E., Wake Forest North Carolina 27587 (US)
(74) Representative: Kirkham, Nicholas Andrew
(86) International application number: PCT/US2001/050338
(87) International publication number: WO 2002/052276

(56) References cited:
- EP-A- 0 406 971
- EP-A- 0 445 626
- EP-A- 0 696 642
- US-A- 5 780 255

## Description

### Field of the Invention

The invention provides a functional protein S assay and methods based on the ability of protein S to prolong the clotting time of plasma in the presence of exogenous Recombinant Tissue Factor, synthetic phospholipids, calcium and either activated protein C or an activator of protein C.

### Background of the Invention

Protein S is a vitamin K-dependent anticoagulant protein which circulates in plasma at a concentration of about 25µg/ml with a half-life of about 2 days. In normal plasma, 60% of protein S binds to C4b-binding protein (C4b-BP) non-covalently in a 1:1 ratio with high affinity. Protein S that is bound to C4b-BP is inactive. The remaining 40% of protein S exists as free protein in plasma and is believed to be the physiologically active anticoagulant form which acts on the cell membrane surface as a cofactor for activated protein C (APC). APC degrades the active forms of procoagulant factors V (FVa) and VIII (FVIIIa) through specific proteolytic cleavage, thereby reducing thrombin generation and prolonging clotting time. Protein S binds to APC and acts as a cofactor and increases the cleavage rate of factors Va and VIIIa. Protein S also exerts a direct inhibitory effect on the prothrombinase complex by binding to factor Xa and to factor Va, and thus impairing prothrombin activation.

Protein S deficiency may be hereditary or acquired. Acquired deficiency may be observed during pregnancy, oral anticoagulant therapy, oral contraceptive use, in liver disease, in newborn infants, as well as in other clinical conditions. Because Protein S is a vitamin K-dependent protein, its concentration decreases during treatment with oral anticoagulants. With a half-life of two days, the rate of decrease for protein S levels is much lower than for protein C and factor VII, which have half-lives of several hours. A representative normal range for total protein S is 70-140%. Considering 25 µg/ml as the mean concentration, this corresponds to a range of 15-35µg/ml. Protein S levels may be influenced by sex hormones such as estrogens. Pre-menopausal women have lower values than men and post-menopausal women. Significantly lower mean values of total and free protein S are found in pregnant women (from 25 µg/ml to 15µg/ml) and women using oral contraceptives (from 25µg/ml to 18 µg/ml). Acquired and congenital protein S deficiency is associated with an increased risk of thrombosis (e.g., deep vein thrombosis) due to a decrease of blood anticoagulant potential. Hereditary protein S deficiencies include familial thrombophilia.

The current subclassification of protein S deficiency into three types was recommended by the Scientific Standardization Committee of the International Society on Thrombosis and Haemostasis (ISTH) in 1992. Type I is characterized by low levels of total and free protein S with a decrease in functional protein S activity. Type II is characterized by normal levels of total and free protein S with a decrease in functional protein S activity. Type III is characterized by normal levels of total protein S and a low level of free protein S, with a decrease in functional protein S activity.

Antigenic (immunological) assays measure the concentrations of either total or free protein S, depending on the antibody and/or procedure used. Functional assays for protein S measure the biological activity of protein S. Since protein S bound to C4BP does not have anticoagulant activity, it is important to know the concentration of the free protein S that is available to act as a cofactor for APC. Free protein S can be quantitatively determined in several ways, for example, the C4BP-protein S complex may be precipitated with polyethylene glycol and the concentration of free protein S in the supernatant may be determined. Alternatively, free protein S may be directly measured by capturing free protein S with immobilized C4BP (e.g., C4BP bound to wells of a microplate) and quantitating with antibody (Coaliza® Protein S-Free Assay, Chromogenix-Instrumentation Laboratory Company SpA, Milan Italy).

Protein S activity does not always correlate with protein S levels in a plasma sample. For example, a free protein S concentration obtained using an antigenic method correlates well with functional activity for patients with Types I and III but not Type II protein S deficiency for a number of reasons. First, antigenic assays measure both fully carboxylated (active) and non-carboxylated (inactive) forms of free protein S. Second, the functional protein S assays are complicated by the presence of both the free and complexed forms in plasma. Thus, antigenic assays can overestimate the level of functional protein S. For example, an antigenic assay of plasma from patients receiving warfarin will give higher values than those obtained using a functional assay. It is therefore important that both a functional and an antigenic assay be performed to screen patients at risk of thrombotic disease for protein S deficiency (i.e., deficient protein S levels and/or deficient protein S activity).

In some functional protein S activity assays, the effect of free protein S as a cofactor to APC is determined. These assays are predominantly coagulometric and measure the prolongation of the clotting time due to free protein S activity as a consequence of the degradation of FVa and FVIIIa by APC. APC-cofactor methods for free protein S activity have traditionally included the prothrombin time (PT), the activated partial thromboplastin time (APTT) and factor Xa-based methods, described below. In addition, free Protein S also exerts an APC-independent anticogulation activity through direct binding to factor Va, factor Xa and factor VIII. An assay of the APC-independent anticoagulant activity of protein S has been developed in which the clotting time is determined in the presence and absence of a polyclonal protein S antibody.

Protein S functional assays may be based on the prothrombin time (PT). The cofactor activity of protein S is confined to the APC-dependent degradation of factors Va and VIIIa. Originally, a method was developed for characterization of purified protein S, which was later followed by a functional test for determining protein S in plasma. (Walker (1984) Sem. Thromb. Hemost. 10:131-38). Protein S activity is determined by mixing a plasma sample with protein S-deficient plasma. The stimulating effect of protein S on the anticoagulant activity of APC is measured by observing clotting time following the addition of thromboplastin (Tissue Factor) and calcium ions to a plasma sample with and without the addition of exogenous APC or exogenous protein C activator (PCA). PCA may be isolated from snake venom from *Agkistrodon contortrix,* which is known under the proprietary name Protac® C (Pentapharm, Basle, Switzerland). A resolution of 40-50 seconds is obtained between 0 and 100% protein S.

Protein S functional assays alternatively can be based on the prolongation of activated partial thromboplastin time (APTT) due to exogenous APC or exogenous PCA.

The standard APTT reaction begins by adding a surface-activating agent (e.g., Kaolin, silica, ellagic acid) and a phospholipid preparation to a plasma sample, thereby achieving maximum activation of factor XI. Calcium is then added to activate the coagulation cascade and the time for clot formation is determined.

In APC resistance assays (e.g., COATEST and COATEST F), two APTT reactions are performed, one in the presence of APC (or PCA) and the other in its absence. The result can be calculated either as a prolongation of clotting time or as a ratio between the clotting times in the presence or absence of APC (or PCA). The APTT reaction without the additional of APC (or PCA) should be within the normal range of 25-40 seconds.

However, the cut-off value for all assays known to date varies between laboratories, instruments, reagent handling and other preanalytical variables. For this reason, APTT and PT assays typically require that a normal control sample be run in parallel. In such cases, the clotting time and/or clotting time prolongation of the patient sample is compared to that of the normal control sample or samples of known protein S content.

Other protein S assays include FXa-based methods, wherein coagulation is triggered by factor Xa in the presence of calcium ions and phospholipids. Originally, undiluted plasma was used. (Comp (1984) J. Clin. Invest. 74:2082-2088.). This was later replaced by methods to minimize interference by prothrombin levels in the plasma, allowing dilution of test plasma and providing close to 100 seconds resolution between 0 and 100% protein S. (Wiesel et al. (1990) Thromb. Res. 58:461-468.) In one variant of the method, free protein S in the test plasma is first adsorbed on an insolubilized monoclonal protein S antibody. (D'Angelo et al. (1988) J. Clin. Invest. 81:1445-1454). Factor Xa has also been used as a trigger in a system utilizing purified components. Dahlback (1986) J. Biol. Chem. 261:12022-12027).

A prothrombin time method is described in U.S. Patent No. 5,726,028. The assay uses Thromborel S®. a tissue factor/phospholipid preparation from human placenta and protein C activator. The endogenous protein C in the sample is activated by the protein C activator and forms with protein S active APC/protein S complexes. Clotting is induced by adding calcium ions, and the resulting APC/protein S complexes delay clot formation.

However, this and other assay (see also EP-A-0 406 971) available generally use crude extracts of tissue factor and phospholipid. In addition, activated protein C, which is also used in the assays is obtained by activating a plasma sample containing protein C with a crude protein C activator, such as snake venom activator, for example. As a consequence of impurities present in these crude reagents, the traditional protein S functional assays suffer from poor reproducibility, low sensitivity and instability.

A need exists, therefore, for a reproducible, sensitive and stable, and functional Protein S assay that, optionally, does not require comparison of the patient results to the results from a normal

### Brief Description of the Drawings

The foregoing and other objects, features and advantages of the present invention, as well as the invention itself, will be more fully understood from the following description of preferred embodiments when read together with the accompanying drawings, in which:
Figure 1 shows an exemplary calibration curve.
Figure 2 shows a comparison between PT measurements obtained using the functional assay and the antigenic assay.

### Summary of the Invention

The invention relates generally to a new functional protein S assay and kit that is based on the ability of endogenous protein S to prolong clotting time in response to exogenous PCA or APC. In the assay procedure, a test plasma sample is diluted with protein S-deficient normal plasma, followed by the addition of recombinant tissue factor (rTF), synthetic phospholipid (sPL) and activated with or without purified or recombinant protein C (pAPC or rAPC) or purified or recombinant protein C activator (pPCA or rPCA) and appropriate salts. The prolongation of clotting time due to exogenous PCA or APC is then determined and is indicative of the protein S activity in the test sample. The prolongation of clotting time obtained for the patient sample may be compared to a standard curve of normal plasma clotting. Insufficient prolongation of clotting time is indicative of protein S deficiency.

The TF is recombinant (e.g., rabbit or human). The TF is preferably re-lipidated with PL prior to adding to the protein S assay reagent.

The PL is sPL. In a preferred embodiment, the PL comprises 1,2-dioleoyl-sn-glycero-3-phosphocholine (PC), 1,2-dioleoyl-sn-glycero-3-phospho-L-serine (PS), and 1,2-dioleoyl-sn-glycero-3-phosphoethanolamine (PE). The PC:PS:PE is preferably in a molar ratio of about 3 to about 4 to about 5.

APC is preferably rAPC. If exogenous APC is used, it is preferably derived by activation of exogenous protein C by proteolysis with a suitable enzyme. Preferred enzymes are those which do not activate or otherwise influence any other factors in the clotting system apart from protein C. Particularly preferred is thrombin. Also preferred are protein C activators from the venom of snakes, such as, for example, *Agkistrodon contortrix contortrix, Agkistrodon bilineatus* or *Agkistroron halys halys.*

In embodiments where clotting time is observed chromogenically, for example, a chromogenic substrate for a component of the coagulation cascade influenced by PS-cofactor activity may be added for thrombin to facilitate chromogenic determination.

The PS-deficient plasma, TF and APC are preferably derived from a mammalian source such as, for example, a cow, pig, rabbit or human.

In another aspect, the invention provides a kit for measuring the functional activity of PS having a container containing PS- deficient plasma and one or more containers comprising rTF; and sPL; and calcium; and APC or PCA. The kit may also comprise calibration plasma for preparing a standard curve or a control plasma sample with a know protein S activity.

### Description of Preferred Embodiments

The invention provides a sensitive functional protein S (PS) assay based on the ability of endogenous protein S to prolong the clotting time in response to exogenous APC or PCA in a PT-based assay. Thus, TF, PL, calcium, and PCA or APC are added to an aliquot of a patient's sample, and clotting times are observed. The clotting time is compared to a standard curve of clotting times of plasma samples having known protein S activities. The use of synthetic PL, recombinant TF and purified activated APC allows for optimization of reagent sensitivity, reproducibility and specificity.

The traditional functional protein S assays normally involve the use of TF derived from brain powder extract and crude PL from plant and animal sources. (U.S. Patent No. 5,726,028). However, endogenous Protein S cannot significantly prolong the clotting time when using these reagents, which are often insensitive to Protein S levels. In the instant invention, the assay reagents are specifically sensitive for measuring protein S activity, and are, therefore, referred to hereinafter collectively as the protein S (PS) reagent.

The contents of a preferred PS reagent and concentration ranges of the reagents are shown in Table 1. The PS reagent contains purified or recombinant Activated Protein C (pAPC or rAPC), synthetic PL (sPL) and recombinant TF (rTF), to avoid lot to lot variation in activity and sensitivity. The assay contains purified APC, rTF and sPL. The use of sPL and recombinant TF avoids contamination from the source (e.g., brain powder), and provides a much easier and more controllable manufacturing process. The amounts of TF and PL in the PS reagent required by the disclosed assays are less than those required for traditional PT assays.

APC may be generated by activating exogenous or endogenous plasma protein C with snake venom activator (e.g., Protac^{®}), which is time-consuming and which may also result in insufficient or variable activation of APC (e.g., from lot to lot). Alternatively, exogenous protein C may be activated using thrombin as described in Example 4.

The PS reagent of the invention preferably contains purified APC to eliminate the external activation step and to simplify the assay. The use of purified APC (pAPC) ensures that APC levels are constant from assay to assay. Suitable pAPC may be purified from any mammalian source such as, for example, human, bovine, porcine, equine and rabbit.

Alternatively, protein C activator (PCA) is used in the assay to activate endogenous protein C. The concentration of PCA is chosen so that a suitable prolongation of the clotting time in the plasma is generated by the exogenous PCA. A suitable prolongation of the clotting time (as compared with the clotting time in the absence of a PCA) is one which, on the basis of the type of apparatus used, allows significant differences from normal plasmas to be detected. The prolongation time is preferably at least about 25%, 50%, or 75%, particularly preferably at least about 100%, or about 200%.

Tissue Factor (TF; also called thromboplastin) is the protein responsible for triggering blood clotting in PT-based assays. It is an integral membrane protein that must be incorporated into phospholipid vesicles for optimal activity. Recombinant TF (rTF) may be obtained from any mammalian source, such as, for example, human, bovine, porcine, equine. Preferred TF is recombinant rabbit TF, such as that described in U.S. Patent Number 5,858,724 or 6,100,072. Recombinant TF may be obtained by *in vitro* transcription and translation, for example. Alternatively, natural purified TF could be used. TF may be purified according to the method provided in Example 2. In a preferred embodiment, the PS reagent is prepared with rTF that has been re-lipidated with sPL.

Synthetic phospholipid (sPL) may be prepared, e.g., by organic synthesis using standard methods. The sPL of the invention is preferably a mixture of three lipids: 1,2-dioleoyl-sn-glycero-3-phosphocholine (PC), 1,2-dioleoyl-sn-glycero-3-phospho-L-serine (PS), and 1,2-dioleoyl-sn-glycero-3-phosphoethanolamine (PE). In a preferred embodiment, the molar ratio of PC:PS:PE is about 3: about 4: about 5.

sPL used for re-lipidation of TF prior to the protein S assay was prepared by an extrusion method. In this method, PLs are forced or extruded through two different membranes (pore sizes 0.45µm and 0.1µm) sequentially and repeatedly forced through a 0.1µm membrane in order to form lipid vesicles or micelles. Alternatively, PL can be treated by a detergent solubilization process, wherein the PLs are dissolved in detergent to form loose lipid vesicles or micelles. Purified or recombinant TF is then added and becomes incorporated into the vesicles. The detergent is then removed, causing the vesicle to contract or shrink, causing the TF to intercalate between PL molecules. The TF is thereby exposed to the exterior of the vesicle.

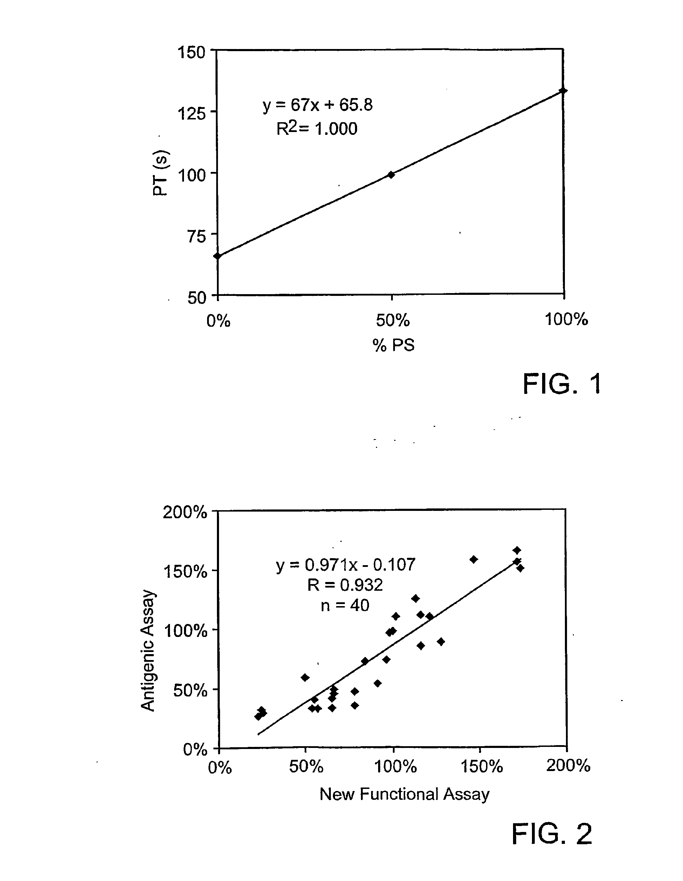

The protein S assay of the invention involves mixing together test plasma, PS deficient plasma, factor diluent and a PS assay reagent comprising TF, PL and APC or PLA (see, for example, Example 5). Potential analytical interferences are minimized by diluting the test sample about 20-fold with PS-deficient plasma and factor diluent, so that the assay is specific for protein S. The assay results are linear over the range of 5%-150% Protein S activity. The variation of calibration curves is small with <3% coefficient of variation (CV) over a period of 2 weeks. The assay is reproducible, with <3% within-run CV and <5% between-run CV for normal samples, and <5% within-run CV and <8% between-run CV for abnormal samples (<30% PS) (Figure 1).

The assay specificity was demonstrated by a good correlation between functional PS and free antigenic PS in normal and patient samples (slope = 0.971, intercept =-0.107, and r = 0.932) (Figure 2). An antigenic assay to determine free PS concentration was performed according to standard methods (e.g., Coaliza). The functional and antigenic protein S assays gave comparable protein S recovery in APC-resistance samples, indicating that APC-resistance does not interfere with the functional assay.

Prolongation of the clotting time may be measured in various ways (e.g., photometrically or chromogenically). When clotting is measured chromogenically, a substrate for a component of the coagulation cascade that is influenced by protein S activity may be added to the assay. An exemplary chromogenic substrate would be a substrate for thrombin (e.g., H-D-Phe-Pip-Arg-pNA-2HCl; MW 625.6; S-2238, Chromogenix).

High sensitivity, specificity, reproducibility and simplicity make this assay suitable for automation on coagulation analyzers (e.g., IL Coagulation or ELECTRA System, Instrumentation Laboratory) according to art known methods, e.g., for screening for congenital and acquired protein S deficiency. In addition, the assay allows the use of calibration curves to determine protein S activity.

### EXEMPLIFICATION

### Example 1: Preparation of Phospholipids by Extrusion

PLs micelles were prepared by extrusion. In this method, PLs are first suspended in a buffered saline solution to give large, multilamellar vesicles. The vesicle solution, e.g., 0.5-1.0 mls, is then passed through a 0.45µm polycarbonate membrane and repeatedly passed through a 0.1µm polycarbonate membrane six times. The result is uniformly sized, unilamellar vesicles, approximately 100 nm in diameter. The extrusion process is performed using, for example a LiposoFast-100 Extruder (Avestin, Inc., Ottawa, Canada). The LiposoFast-100 is a medium pressure extruder that uses compressed gas (e.g., nitrogen) at up to 600 PSI to pressurize the sample cylinder and force the starting material through the membrane. The extruded PL is then added to TF, which attaches to the outside of the lipid vesicle.

Extrusion may be performed according to standard methods or according to the manufacturer's recommendations, e.g., the method of http://tf7.org/methods.html - James H. Morrissey, Dept. of Biochemistry, University of Illinois at Urbana-Champaign, Urbana, IL 61801, USA, as follows:
1. Dispense 2.6 µM total phospholipids (PL) in a glass test tube
2. Using a fume hood, dry the PL mixture under a gentle stream of nitrogen or argon. When dry, speed-vac for an additional 60 minutes under high vacuum to remove any residual chloroform.
3. To the dried PL, add 2.6 ml room temperature HBS solution and cover the end of the tube with parafilm. Let sit 1 hr at room temperature.
4. Vortex tube vigorously to completely resuspend the PL. The result should be a milky, uniform suspension. You can aid the process of resuspension by freezing and thawing the suspension multiple times (as many as ten times).
5. Load 0.5 ml of the lipid suspension into one of the two glass syringes (containing a 0.45µm filter) of the Lioposofast machine and attach it to the Luer lock on one side of the device. Close the other (empty) syringe and attach it to the Luer lock on the opposite side of the device.
7. Press the loaded syringe to pass its entire contents through the filter and into the opposing syringe. Change the 45µm to a 0.1µm Repeat this process alternately with the two syringes for a total of at least 7 passes. It is essential that you always use an odd number of passes, so that the final product will end up in what was originally the empty syringe. This will ensure that none of the starting multilamellar vesicles will contaminate the final product.
8. Remove the final product and repeat steps 6 and 7 for the remaining, unprocessed phospholipid suspension, until all of the suspension has been processed.
9. Store the final product at 4°C. The result is a uniform suspension of unilamellar vesicles (about 100 nm in diameter) containing a total of 1 mM phospholipid in HBS.

### Example 2: Purification of TF from Cell Lysates

Tissue factor (TF) is purified from cell lysates using the following method. Cells producing TF are washed with TBS and resuspended to 2x10⁷ /ml in TBS containing 0.25% Triton-X100, 10 µg/ml soybean trypsin inhibitor, and 1 mM EDTA. After incubation for 30 min at 4°C., the cellular debris is removed by centrifuging for 20 min at about 5000x g at 4°C. The clarified lysate is diluted 2.5-fold with TBS to reduce the Triton concentration to 0.1% and passed through an immunoaffinity resin containing a covalently coupled monoclonal antibody directed against TF. The resin bed is washed with 2 to 3 bed volumes of TBS+0.1% Triton-X100, 2 to 3 volumes 20 mM Tris, pH 7.5, 0.5 M NaCl, 0.1 % Triton-X100, and finally 2 to 3 bed volumes 0.5 M NaCl, 0.1 % Triton-X100. The bound protein is eluted from the resin with 0.1 M glycine, pH 2.5, 0.1 % Triton-X100. Fractions collected after the buffer was changed to glycine are neutralized immediately with an appropriate volume of 1 M Tris, pH 8. TF is found in those fractions immediately surrounding the point where the pH of the column effluent changes. The fractions containing TF are pooled, dialyzed against 20 mM Tris, pH 8, 0.1% Triton-X100, and concentrated by binding the TF to a small bed volume DEAE Trisacryl column (IBF Biotechniques, Columbia, Md.). The Triton-X100 is replaced with CHAPS (Calbiochem.) by washing the resin bed with at least 10 bed volumes of 20 mM Tris, pH 8 containing 10 mM CHAPS. The TF is eluted with a single step of 0.5 M NaCl in 20 mM Tris, pH 8, 10mM CHAPS.

### Example 3: Re-lipidation of Tissue Factor

A preferred re-lipidation process is as follows: 66g of sPL is reconstituted with 4.4ml of 100mM CHAPS in buffer. The sPL was mixed at 30-37°C until completely dissolved. The PL was transferred into a jacketed, PVDF-coated vessel and the lipid container rinsed with 2X volume (400ml) buffer. 100ml 20mM CHAPS/BGG was added to the PVDF-coated vessel and mixed at 200-400 RPM for 5-10 min., avoiding excess foaming. Recombinant TF was quick thawed and was added to the PL. The remaining buffer was added to the TF/PL mixture. The TF/PL mixture was incubated for 55-65 min. at 27-33°C with an overhead mixer at 200-400 RPM. XAD-6 resin was washed with buffer and aliquoted into 6 aliquots. One aliquot of the resin was vacuum-filtered and added to the TF/PL mixture. The TF/PL mixture was incubated with mixing using an overhead mixture at 200-400 RPM for 2 hours +/- 15 min. at 27-33°C. Additional aliquots of resin were added to the TF/PL mixture. After the addition of the 4^{th} aliquot, the TF/PL mixture remained mixing overnight at 27-33°C. At day 3, the remaining aliquots of resin were added and the TF/PL mixture was filtered through a series of 250 µM NYTEX Mesh, 2-10 and 0.22µM filters and mixed for 15 min. 4L of dilution buffer was added to 1L undiluted TF/PL mixture and mixed for 15 min.

### Example 3: Tissue Factor Relipidation Using Detergent

This technique for incorporating TF into PL vesicles uses the dialyzable, nonionic detergent, *n*-octyl-beta-D-glucopyranoside (octylglucoside) (Calbiochem Corp., La Jolla, CA). (http://tf7.org/methods.html; Neuenschwander et al. (1993) J. Biol. Chem. 268:21489-21492) (see also U.S. Patent No. 6,203,816, the contents of which are incorporated herein by reference).

In this method, PLs and TF are both dissolved in octylglucoside, forming mixed micelles. Since octylglucoside has a high critical micelle concentration (CMC = 20 to 25 mM), it can readily be removed from solutions by dialysis. As the octylglucoside dialyses out, the phospholipids organize into unilamellar vesicles. TF becomes embedded in these vesicles by virtue of its single membrane-spanning domain, located near the C-terminus of the protein. Typically, about 50 to 80% of the TF molecules face outward in these vesicles. The remaining TF molecules face inward and are therefore unable to interact with factor VII/VIIa. (Neuenschwander et al. (1993) J. Biol. Chem. 268:21489-21492). To obtain relipidated TF that is not contaminated with detergent, it is preferable to use TF stock solutions that contain a dialyzable detergent like CHAPS or octylglucoside, rather than Triton. PLs in aqueous solution are subject to oxidation. For this reason, once TF has been relipidated it should typically be used within about 2 or 3 weeks. (For some applications, older TF preparations can still be used with good results. Be aware, though, that such preparations may contain oxidized phospholipids.)

For most applications, TF activity is maximal when vesicles contain 20 mol% phosphatidylserine or less, so there is normally no reason to exceed this level. Note that soluble tissue factor (sTF) cannot be incorporated into phospholipids; in which the membrane spanning domain is intact should be used. Blank vesicles can be made simply by leaving out the TF in the protocol.

### Preparation of phospholipid solution in octylglucoside

1. For each sample, dispense 2.6 µM total PLs in a glass test tube, using the desired polar ratio of PL (e.g., 30% PC, 40% PS, 50% PE) (Avanti Polar Lipid, Alabaster, Alabama).
2. Dry the PL mixture under a gentle stream of argon or nitrogen. If possible, set the tube at an angle so the PLs form a thin film on the side of the tube.
3. When the tube appears dry, speed-vac for an additional 60 minutes under high vacuum to ensure that residual chloroform is removed.
4. To the tube of dried-down PLs, add 400µl freshly prepared OG/HBS solution (100 mM *n*-octyl-beta-D-glucopyranoside in HBS (100 mM NaCl, 20 mM Hepes/NaOH buffer, pH 7.5, 0.02% (w/v) sodium azide (RT))). Vortex vigorously to completely dissolve the dried-down PLs.
   Relipidation procedure
5. To the tube containing 400µl of PL/octylglucoside solution, add the desired amount of membrane TF (preferably, dissolved in CHAPS or octylglucoside) and enough HBSA (HBS with 0.1% (w/v) bovine serum albumin) to make the final volume 1 ml. A typical molar ratio of PL to TF is 8700:1, ratios as high as 50,000:1 and as low as 3,000:1 may be used. The final volume will be 1 ml.
6. Mix well and incubate the sample for 30 min at room temperature (RT).
7. Dialyze the sample at RT against three changes of HBS (24 hr each, for a total of 72 hr). Store the final product at 4°C.

The final product is about 1 ml of relipidated TF containing approximately 2.6 mM phospholipid. Because the recovery from dialysis may not be 100%, these amounts are only approximate. Precise concentrations of available TF and total PL can be determined by performing an analysis of exposed TF (titrate with factor VIIa by measuring the TF-induced increase in VIIa amidolytic activity), and an analysis of PL content. (Neuenschwander et al.).

### Example 4: Preparation of Activated Protein C

In a preferred embodiment, APC is derived by activation of protein C with thrombin according to standard methods. For example, a frozen protein C fraction from human placenta (Pharmacia UpJohn) is filtered and affinity purified using an Affigel column to which HPC-4 monoclonal antibody (specific for human protein C) (Instrumentation Laboratory Company) is bound. The affinity purified PC is eluted from the Affigel HPC-4 column and is ultrafiltered again. SP Sephadex C-50 purified thrombin is added to the purified PC to activate the PC (APC). The APC is passed through a SP-Sephadex C-50 to remove the thrombin. CaCl₂ and BSA are added to the eluate containing purified APC.

### Example 5: The Protein S Assay

Human plasma samples were tested for Protein S activity as compared to a standard curve. The assay was performed as follows: Nine parts freshly drawn venous blood was collected into one part trisodium citrate and red cells removed by standard methods. 4µl of the blood plasma sample is mixed with 25µl of PS deficient plasma (1.0ml human plasma which has been artificially depleted of protein S), lyophilized and resuspended in 1.0 ml H₂O), 51µl of factor diluent (0.85% sodium chloride, 0.1 % sodium azide and 80µl of PS assay reagent (15mM HEPES, free acid, 18mM HEPES sodium, 5g/l bovine serum albumin, 140mM sodium chloride, 10mM calcium chloride, 0.0067% sodium omadine, 50µM ciprofloxacin, 0.0667% polybrene, 300ng/l recombinant rabbit tissue factor, 12.5µM synthetic phospholipid (PC/PS/PE 3:4:5, e.g., 9.66 µM PC, 12.9 µM PS, 16.1 µM PE), 4mg/l activated human protein C; pH7.5) and the clotting time measured using a coagulation instrument or a spectrophotometer.

Any of a number of coagulation instruments may be used to perform the test and measure clotting time, (e.g., the ACL, ACL Futura, or ELECTRA; Instrumentation Laboratory Company, Lexington, Massachusetts). Depending on the type of machine used, a calibration curve may be generated and used for measuring a number of samples before another calibration curve must be generated. The instrument is programmed to make a calibration curve from various mixtures of calibration plasma (plasma in which the coagulation factor levels are known and which contains about 100% protein S) and protein S-deficient plasma (containing about 0% protein S). The two solutions act as the two end points of the curve and intermediate points on the curve are generated by mixing different relative amounts of the two plasmas and measuring their clotting time. For example, serial dilutions of calibration plasma with protein S-deficient plasma may generate plasma samples with about 10%, about 20%, about 30%, about 40%, about 50%, about 60%, about 70%, about 80% or about 90% protein S activity. Once the calibration curve samples have been measured, the clotting times vs. protein S concentration is graphed. The clotting times of test samples are then measured and read against the curve to obtain protein S activity. As a quality control measure, protein S control plasma with a pre-determined protein S activity is run along side the samples to ensure the assay is performing accurately.

Data analysis is performed according to instrumentation specifications. For example, using an ACL, ACL Futura or ELECTRA instrument, results are reported automatically by the instrument as % activity. Each laboratory must establish there own normal range. For an ACL Futura or ELECTRA instrument, once a calibration run is complete and a standard curve is generated, the instrument will store the calibration for future patient runs.

The optimized concentrations and suitable concentration ranges of the PS reagent ingredients are shown in Table I. To avoid possible influence of Factor V Leiden mutation (APC-R) on the actual values, patient samples with results outside the normal range should be manually diluted 1:2 with Protein S deficient plasma and re-assayed. The result is then multiplied by 2.

**Table I: Optimal Concentrations and Concentration Ranges of Assay Reagents**

| ***Materials*** | ***Optimized Concentration*** | ***Concentration Range*** |
|---|---|---|
| HEPES Free Acid | 15 mM | 10-20 mM |
| HEPES Sodium Salt | 18mM | 10-25 mM |
| Sodium Chloride | 140 mM | 130-150 mM |
| Calcium Chloride | 10 mM | 8-12 mM |
| Sodium Omadine | 0.0067% | 0.0040-0.0100% |
| Ciprofloxacin | 50µM | 30-100 µM |
| Polybrene | 0.667% | 0.600-1.00% |
| BSA | .5% | 3-7.5 g/L |
| sPL | 12.5 µM | 10.0-15.0 µM |
| APC | 4 mg/L | 3-5 mg/L |
| rTF | 0.3mg/L | 0.8-1.2 µg/L |
| pH | 7.5 | 7.45∼7.65 |
| | | |

An exemplary calibration or standard curve is shown in Figure 1. A patient's plasma sample was tested and a functional protein S level was read from the calibration curve by comparing the coagulation time of the patient sample to the value on the curve. The prolongation of the clotting time was proportional to the protein S activity in the test sample.

A comparison of the performance of three coagulation instruments is shown in Table II. In this experiment, normal control plasma was run against protein S control plasma and the % protein S determined both within and between runs. Correlation between the ACL, ACL Futura and ELECTRA systems showed a slope of 1.01,1.02 and 1.03, respectively. All three machines achieved linearity for PS activity between 10% and 150%. These results demonstrate the precision and reproduciability of the assay.

**Table II**

| ACL | Means (% PS) | CV% (Within run) | | CV% (Between run) |
|---|---|---|---|---|
| Normal Control | 95.0 | 2.6 | | 3.1 |
| Protein S Control | 32.3 | 2.5 | | 3.8 |
| | | | | |

| ACL Futura | Mean (% PS) | CV% (Within run) | | CV% (Between run) |
|---|---|---|---|---|
| Normal Control | 93.8 | 3.6 | | 4.5 |
| Protein S Control | 31.1 | 4.1 | | 7.3 |
| | | | | |

| ELECTRA | Mean (% PS) | CV% (Within run) | | CV% (Between run) |
|---|---|---|---|---|
| Normal Control | 90.9 | 1.4 | | 4.1 |
| Protein S Control | 27.2 | 2.1 | | 6.4 |
| | | | | |

| Correlation: System | slope | intercept | r | Reference method |
|---|---|---|---|---|
| ACL | 1.01 | -5.883 | 0.982 | IL Clotting Protein S on ACL |
| ACL Futura | 1.02 | -4.890 | 0.984 | IL Clotting Protein S on ACL |
| ELECTRA | 1.01 | -6.614 | 0.986 | IL Clotting Protein S on ACL |
| The precision and correlation results were obtained using specific lots of reagents and controls. | | | | |

| Linearity: System | | | | |
|---|---|---|---|---|
| ACL, ACL Futura and ELECTRA | | 10-150 (% PS activity) | | |

Table III shows a comparison of the methods of the invention to immunoglobulin assays for plasma samples from patients with various diseases. Column 2 shows the protein S assay of the invention performed on an ACL3000 instrument. Column 3 shows instant protein S assay of the invention performed on a Futura instrument. Column 4 shows a protein S assay (utilizing bovine TF) performed on an ACL3000 instrument. Column 5 shows the results using a IL Test™ Free Protein S kit (Latex-immunological). Column 6 shows the results using a Coaliza® test kit. Column 7 shows the difference between the values obtained in Column 3 minus Column 2. Column 8 shows the differences in values obtained in Column 5 minus Column 2. Column 10 shows the difference between the values obtained for Column 6 minus Column 2. Column 11 shows the difference between the values in Column 6 minus Column 5.

**Table III Comparison of the Protein S assay to the Bovine TF, FPS (Latex) and ELISA assays on various coagulation machines.**

| | **Disease state** | **ProS (ACL3000)** | **ProS (Futura)** | **Bov (ACL3000)** | **FPS (Latex)** | **ELISA** | **ProS(Futura) Minus New (ACL3000)** | **Bovine Minus New (ACL3000)** | **rPS(Latex) Minus New (ACL3000)** | **ELISA Minus New (ACL3000)** | **ELISA% Minus FPS (Latex)** |
|---|---|---|---|---|---|---|---|---|---|---|---|
| 1 | PS Type II 1(11) | 26% | 25% | 30% | 29% | 33% | -1% | 4% | 3% | 7% | 4% |
| 2 | PS Type11 3 | 23% | 23% | 30% | 27% | 26% | 0% | 7% | 4% | 3% | -1% |
| 3 | PS def 6 | 25% | 24% | 36% | 32% | 32% | -1% | 11% | 7% | 7% | 0% |
| 4 | PS/PC 10 | 65% | 67% | 68% | 42% | 50% | 2% | 3% | -23% | -15% | 8% |
| 5 | PS/PC 9 | 78% | 65% | 73% | 47% | 55% | -13% | -5% | -31% | -23% | 8% |
| 6 | PS/PC 7 | 57% | 48% | 50% | 33% | 36% | -9% | -7% | -24% | -21% | 3% |
| 7 | PS/PC 8 | 55% | 51% | 60% | 41% | 46% | -4% | 5% | -14% | -10% | 5% |
| 8 | Liver 1 | 66% | 61% | 78% | 50% | 62% | -5% | 12% | -16% | -4% | 12% |
| 9 | Liver 2 | 121% | 111% | 117% | 110% | 104% | -10% | -4% | -11% | -17% | -6% |
| 10 | OAC 12 | 66% | 55% | 65% | 46% | 52% | -11% | -1% | -20% | -14% | 6% |
| 11 | Heprin 12 | 96% | 88% | 96% | 74% | 85% | -8% | 0% | -22% | -11% | 11% |
| 12 | Heprin 13 | 128% | 116% | 127% | 89% | 97% | -12% | -1% | -39% | -31% | 7% |
| 13 | OAC 11 | 78% | 62% | 83% | 36% | 40% | -16% | 5% | -43% | -38% | 5% |
| 14 | OAC 13 | 54% | 45% | 51% | 33% | 33% | -9% | -3% | -21% | -21% | 0% |
| 15 | OAC 14 | 65% | 53% | 61% | 33% | 37% | -12% | -4% | -32% | -29% | 3% |
| 16 | OAC 15 | 91% | 78% | 86% | 54% | 63% | -13% | -5% | -37% | -29% | 9% |
| 17 | PS high 7 | 174% | 162% | 172% | 151% | 168% | -12% | -2% | -23% | -6% | 17% |
| 19 | PS high 6 | 172% | 172% | 174% | 156% | 172% | 0% | 2% | -16% | 0% | 16% |
| 20 | PS high 10 | 172% | 160% | 178% | 166% | 154% | -12% | 6% | -6% | -18% | -12% |
| 22 | PS high 9 | 147% | 150% | 142% | 158% | 162% | 3% | -5% | 11% | 15% | 4% |
| 24 | GKN.P.1 | 84% | 72% | 79% | 73% | 80% | -12% | -5% | -12% | -4% | 8% |
| 25 | GKN.P.2 | 50% | 43% | 58% | 59% | 60% | -7% | 8% | 9% | 10% | 1% |
| 26 | GKN.P.3 | 116% | 93% | 107% | 86% | 85% | -23% | -9% | -30% | -31% | -1% |
| 27 | APCR 241 | 102% | 89% | 100% | 110% | 110% | -13% | -2% | 8% | 8% | 0% |
| 30 | APCR 24132 | 100% | 95% | 96% | 98% | 109% | -5% | -4% | -2% | 9% | 11% |
| 34 | APCR 23974 | 98% | 85% | 96% | 97% | 106% | -13% | -2% | -1% | 8% | 9% |
| 37 | APCR 23976 | 113% | 96% | 112% | 125% | 122% | -17% | -1% | 12% | 9% | -4% |
| 40 | APCR 23969 | 116% | 99% | 118% | 112% | 125% | -17% | 2% | -4% | 9% | 14% |

### Equivalents

The foregoing embodiments are therefore to be considered in all respects illustrative rather than limiting of the invention described herein.

## Claims

1. A method for measuring protein S activity in a plasma sample, comprising the steps of:
(a) mixing a sample of test plasma with protein S-deficient plasma, recombinant tissue factor, synthetic phospholipid, and calcium, and either:
i) activated protein C, or
ii) an activator of protein C;
measuring the clotting time of the sample; and
(b) comparing the measurement in (a) to a standard curve derived from the clotting time of plasma samples having a range of known protein S activities.

2. A method as claimed in claim 1, wherein the standard curve is prepared by mixing plasma samples having a range of protein S activities with protein S-deficient plasma, recombinant tissue factor, synthetic phospholipid, calcium, and either:
i) an activated protein C, or
ii) an activator of protein C; and
measuring the clotting time and plotting clotting time versus protein S activity.

3. The method claimed in claim 1 or claim 2, wherein the recombinant tissue factor is rabbit tissue factor.

4. The method claimed in any preceding claim, wherein the synthetic phospholipid comprises 1,2-dioleoyl-sn-glycero-3-phosphocholine (PC), 1,2-dioleoyl-sn-glycero-3-phospho-L-serine (PS) and 1,2-dioleoyl-sn-glycero-3-phophoethanolamine (PE).

5. The method claimed in any preceding claim, wherein the molar ratio of PC:PS:PE is about 3 to about 4 to about 5.

6. The method claimed in claim 1 or claim 2, wherein the activated protein C was activated either by thrombin or snake venom.

7. The method claimed in any preceding claim, wherein the activated protein C is derived from recombinant protein C.

8. The method claimed in any preceding claim, wherein one or more of the protein S-deficient plasma, recombinant tissue factor, and APC are derived from a mammalian source selected from the group consisting of a cow, a pig, a rabbit, and a human.

9. The method claimed in any preceding claim, wherein the variation of calibration curves has a <3% coefficient of variation (CV) over a period of either:
i) 2 hours; or
ii) 8 hours; or
iii) 2 weeks.

10. The method claimed in any preceding claim, wherein the assay has a <3% within-run coefficient of variation (CV).

11. The method claimed in any preceding claim, wherein the measuring step is chromogenic or spectrophotometric.

12. The method claimed in any preceding claim further comprising the step of measuring the clotting time of a normal control plasma sample with known protein S activity and comparing that clotting time to the clotting time in step (a) or to the standard curve in step (b).

13. A kit for measuring the functional activity of protein S in a plasma sample, said kit comprising one or more containers containing protein S-deficient plasma, recombinant tissue factor, synthetic phospholipid, calcium, and either;
i) activated protein C or
ii) protein C activator.

14. The kit claimed in claim 13 further comprising calibration plasma comprising about 100% protein S for preparing a standard curve.

15. The kit claimed in claim 13 or claim 14 further comprising normal control plasma between 40-50% protein S.

## Patentansprüche

1. Verfahren zur Messung der Protein S-Aktivität in einer Plasmaprobe, welches die Schritte umfasst von:
(a) Mischen einer Probe von dem Testplasma mit Protein S-Mangelplasma, rekombinantem Gewebefaktor, synthetischem Phospholipid und Calcium und entweder
i) aktiviertem Protein C, oder
ii) einem Aktivator für Protein C;
Messen der Gerinnungszeit von der Probe; und
(b) Vergleichen der Messung in (a) mit einer Standardkurve, die für die Gerinnungszeit von Plasmaproben erhalten wurde, die ein Spektrum bekannter Protein S-Aktivitäten aufweisen.

2. Verfahren nach Anspruch 1, wobei die Standardkurve hergestellt ist durch Mischen von Plasmaproben, welche ein Spektrum von Protein S-Aktivitäten aufweisen, mit Protein S-Mangelplasma, rekombinantem Gewebefaktor, synthetischem Phospholipid und entweder:
i) einem aktiviertem Protein C, oder
ii) einem Aktivator für Protein C; und
Messen der Gerinnungszeit und Erstellen einer grafischen Darstellung von Gerinnungszeit gegen Protein S-Aktivität.

3. Verfahren nach Anspruch 1 oder 2, wobei der rekombinante Gewebefaktor ein Kaninchen-Gewebefaktor ist.

4. Verfahren nach einem der vorangehenden Ansprüche, wobei das synthetische Phospholipid 1,2-Dioleoyl-sn-glycero-3-phosphocholin (PC), 1,2-Dioleoyl-sn-glycero-3-phospho-L-serin (PS) und 1,2-Dioleoyl-sn-glycero-3-phosphoethanolamin (PE) umfasst.

5. Verfahren nach einem der vorangehenden Ansprüche, wobei das Molverhältnis PC:PS:PE ungefähr 3 zu ungefähr 4 zu ungefähr 5 ist.

6. Verfahren nach Anspruch 1 oder 2, wobei das aktivierte Protein C entweder durch Thrombin oder durch Schlangengift aktiviert wurde.

7. Verfahren nach einem der vorangehenden Ansprüche, wobei das aktivierte Protein C von einem rekombinanten Protein C abgeleitet ist.

8. Verfahren nach einem der vorangehenden Ansprüche, wobei eines oder mehrere von dem Protein S-Mangelplasma, dem rekombinantem Gewebefaktor und APC von Säugetieren stammen, die ausgewählt sind aus der Gruppe, die aus einer Kuh, einem Schwein, einem Kaninchen und einem Menschen besteht.

9. Verfahren nach einem der vorangehenden Ansprüche, wobei die Variation der Kalibrierungskurven einen Variationskoeffizienten (VK) von <3 % über einen Zeitraum von entweder
i) 2 Stunden; oder
ii) 8 Stunden; oder
iii) 2 Wochen aufweist.

10. Verfahren nach einem der vorangehenden Ansprüche, wobei der Test einen Variationskoeffizienten (VK) in der Serie von <3 % aufweist.

11. Verfahren nach einem der vorangehenden Ansprüche, wobei der Messschritt chromogen oder spektrophotometrisch ist.

12. Verfahren nach einem der vorangehenden Ansprüche, das ferner den Schritt der Messung der Gerinnungszeit von einem normalen Kontrollplasma mit einer bekannten Protein S-Aktivität und den Vergleich von dieser Gerinnungszeit mit der Gerinnungszeit in Schritt (a) oder mit der Standardkurve in Schritt (b) umfasst.

13. Kit zur Messung der funktionellen Protein S-Aktivität in einer Plasmaprobe, wobei das Kit einen oder mehrere Behältnisse umfasst, die Protein S-Mangelplasma, rekombinanten Gewebefaktor, synthetisches Phospholipid, Calcium und entweder
i) aktiviertes Protein C, oder
ii) Protein C-Aktivator enthalten.

14. Kit nach Anspruch 13, das ferner Kalibrierungsplasma umfasst, das ungefähr 100 % Protein S zur Herstellung einer Standardkurve enthält.

15. Kit nach Anspruch 13 oder 14, das ferner Normal-Kontrollplasma mit zwischen 40 - 50 % Protein S umfasst.

## Revendications

1. Procédé de mesure de l'activité de la protéine S dans un échantillon de plasma, comprenant les étapes consistant à :
(a) mélanger un échantillon de test plasmatique avec un plasma déficient en protéine S, un facteur tissulaire recombinant, un phospholipide synthétique, du calcium et :
i) soit une protéine C activée,
ii) soit un activateur de protéine C ;
en mesurant le temps de coagulation de l'échantillon ; et
(b) comparer la mesure faite en (a) à une courbe étalon dérivée du temps de coagulation d'échantillons de plasma ayant une plage d'activités de la protéine S connues.

2. Procédé selon la revendication 1, dans lequel la courbe étalon est préparée en mélangeant des échantillons de plasma ayant une plage d'activités de la protéine S avec un plasma déficient en protéine S, un facteur tissulaire recombinant, un phospholipide synthétique, du calcium et :
i) soit une protéine C activée, ou
ii) soit un activateur de protéine C ; et
en mesurant le temps de coagulation et en traçant la courbe du temps de coagulation en fonction de l'activité de la protéine S.

3. Procédé selon la revendication 1 ou la revendication 2, dans lequel le facteur tissulaire recombinant est un facteur tissulaire de lapin.

4. Procédé selon l'une quelconque des revendications précédentes, dans lequel le phospholipide synthétique comprend la 1,2-dioléoyl-sn-glycéro-3-phosphocholine (PC), la 1,2-dioléoyl-sn-glycéro-3-phospho-L-sérine (PS) et la 1,2-dioléoyl-sn-glycéro-3-phosphoéthanolamine (PE).

5. Procédé selon l'une quelconque des revendications précédentes, dans lequel le rapport molaire PC:PS:PE se situe dans la plage d'environ 3 sur environ 4 sur environ 5.

6. Procédé selon la revendication 1 ou la revendication 2, dans lequel la protéine C activée a été activée soit par la thrombine soit par du venin de serpent.

7. Procédé selon l'une quelconque des revendications précédentes, dans lequel la protéine C activée est dérivée d'une protéine C recombinante.

8. Procédé selon l'une quelconque des revendications précédentes, dans lequel un ou plusieurs parmi le plasma déficient en protéine S, le facteur tissulaire recombinant et l'APC sont dérivés d'une source mammifère choisie dans le groupe constitué par un bovin, un porc, un lapin et un humain.

9. Procédé selon l'une quelconque des revendications précédentes, dans lequel la variation des courbes d'étalonnage a un coefficient de variation (CV) < 3 % sur une durée :
i) soit de 2 heures,
ii) soit de 8 heures,
iii) soit de 2 semaines.

10. Procédé selon l'une quelconque des revendications précédentes, dans lequel le test a un coefficient de variation (CV) intra-essai < 3%.

11. Procédé selon l'une quelconque des revendications précédentes, dans lequel l'étape de mesure se fait par chromogénie ou spectrophotométrie.

12. Procédé selon l'une quelconque des revendications précédentes, comprenant en plus l'étape consistant à mesurer le temps de coagulation d'un échantillon de plasma de contrôle normal ayant une activité de la protéine S connue et à comparer ce temps de coagulation au temps de coagulation de l'étape (a) ou à la courbe étalon de l'étape (b).

13. Kit de mesure de l'activité fonctionnelle de la protéine S dans un échantillon de plasma, ledit kit comprenant un ou plusieurs récipients contenant un plasma déficient en protéine S, un facteur tissulaire recombinant, un phospholipide synthétique, du calcium et :
i) soit une protéine C activée,
ii) soit un activateur de protéine C.

14. Kit selon la revendication 13, comprenant en plus un plasma d'étalonnage contenant environ 100% de protéine S pour préparer une courbe étalon.

15. Kit selon la revendication 13 ou la revendication 14, comprenant en plus un plasma de contrôle normal contenant de 40 à 50% de protéine S.
